# EUROPÄISCHE PATENTANMELDUNG

(11) **EP 0 936 460 A2**
(43) Veröffentlichungstag der Anmeldung: **18.08.1999**
(21) Anmeldenummer: 99100533.1
(22) Anmeldetag: 13.01.1999
(51) Int. Cl.: G01N 23/04, G01N 23/06

(54) **Formkörper und Verfahren zur semiquantitativen Dichtebestimmung von Knochen, Knochenersatzmaterial und Knochenimplantaten**

(30) Priorität: 23.01.1998 DE 19802414
(71) Anmelder: MERCK PATENT GmbH, 64293 Darmstadt (DE)
(72) Erfinder: Dard, Michel, Dr., 64347 Griesheim (DE); Le Gall, Michel, 13400 Aubagne (FR)

(57) **Zusammenfassung**

Die Erfindung betrifft einen Formkörper mit mindestens zwei parallelen Oberflächen zur semiquantitativen Dichtebestimmung von Knochen, Knochenersatzmaterial und Knochenimplantaten bestehend aus drei oder vier verschiedenen Materialien unterschiedlicher Dichte. Gegenstand der Erfindung ist weiterhin ein Verfahren zur Durchführung der semiquantitativen Dichtebestimmung.

## Beschreibung

Gegenstand der vorliegenden Erfindung ist ein Formkörper mit mindestens zwei parallelen Oberflächen zur semiquantitativen Dichtebestimmung von Knochen, Knochenersatzmaterial und Knochenimplantaten sowie das Verfahren zur Durchführung dieser Dichtebestimmung.

Unabhängig davon, was für radiologische Methoden benutzt werden um menschliches Gewebe mit Knochenersatzmaterial bzw. Knochenimplantaten zu untersuchen, ist es heutzutage nicht möglich, die entstehenden Röntgenaufnahmen einer einfachen Dichtekontrolle zu unterziehen. Ein Vergleich der Röntgenphotos untereinander ist nicht möglich, da unterschiedliche Farbnuancen in den einzelnen Photos, hervorgerufen durch Aufnahmen an mehreren Tagen, zu Fehlinterpretationen führen können.
Desweiteren ist die Anwendung von Bildanalyse-Systemen (Software) dadurch erschwert, daß keine stabile Vorrichtung als Dichtekontrolle im Strahlenfeld verwendet wird.

Aus dem Stand der Technik sind vergleichbare Dichtekontroll-Systeme bekannt. In der FR- 8209057 wird ein Material als Referenz beschrieben, welches Knochengewebe simuliert und in der Radiodensitometrie Anwendung findet. Dieses Material besteht aus 0-55 Gew.% Hydroxylapatit und 45-100 Gew.% Copolymer bestehend aus Polyester und Ethylen.
Nachteilig an diesem Material ist die Tatsache, daß es sich nicht zur Untersuchung von menschlichem Gewebe eignet, welches mit Biomaterialien implantiert ist. Es beschränkt sich auf die Dichtekontrolle von natürlichem Knochenmaterial (Hydroxylapatit), speziell in der Knochenpathologie, um den Verlauf der Knochenaufweichung (Osteoporose) zu verfolgen.

Aufgabe der vorliegenden Erfindung ist es, nunmehr einen Formkörper mit mindestens zwei parallelen Oberflächen bestehend aus Materialien unterschiedlicher Dichte bereitzustellen, durch die es auf einfache Weise möglich ist, semiquantitative Dichtebestimmungen von Knochen, Knochenersatzmaterial und Knochenimplantaten durchzuführen.

Diese Aufgabe wird dadurch gelöst, daß ein Formkörper mit mindestens zwei parallelen Oberflächen zur Verfügung gestellt wird, der aus drei oder mehr verschiedenen Materialien unterschiedlicher Dichte besteht. Bevorzugt sind drei oder vier Materialien. Mit Hilfe dieses Formkörpers lassen sich die radiologischen Veränderungen einer Knochendichte in einem Zeitabschnitt sehr präzise bestimmen. Jede Veränderung des radiologischen Bildes, die auf die physiko-chemischen Eigenschaften zurückzuführen ist, wird durch den Formkörper festgestellt, wodurch jegliche ungenaue oder falsche Interpretation vermieden wird.

Der Formkörper dient zum Kalibrieren aller existierenden oder zukünftigen Bildanalyse-Systemen. Er ist insbesondere zur Untersuchung von harten Geweben (z.B. normaler oder Osteoporose-Knochen), die mit Biomaterialien (z.B. Hip-Prothese, Knochenersatz, Zahnimplantate, Zement) implantiert sind, geeignet.

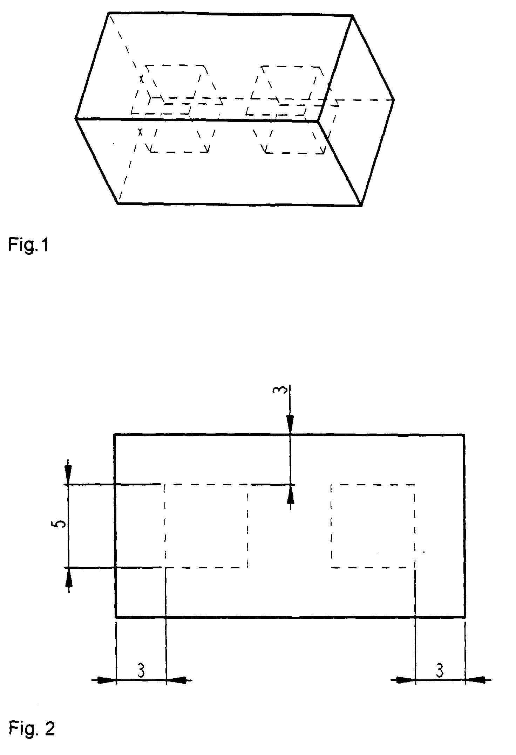

Der Formkörper mit parallelen, vorzugsweise planparallelen Oberflächen, umfaßt 2 oder mehr Referenzkörper mit 2-20 mm, vorzugsweise 5 mm, Seitenlänge (siehe Fig. 1) aus Materialien mit unterschiedlichen Dichten. Bevorzugt enthält der Formkörper 2 oder mehr Referenzkörper. Die Referenzkörper können, wie dem Fachmann bekannt, verschieden ausgeführt sein. Bevorzugt sind die Referenzkörper so gestaltet, daß sie mindestens zwei parallele Oberflächen besitzen, die bevorzugt parallel zu den beiden parallelen Oberflächen des Formkörpers orientiert sind. Als besonders geeignet haben sich Referenzkörper in Form von Würfeln, Quadern, Rhomboedern, Prismen und Zylindern erwiesen.
Der erste Referenzkörper besteht aus einem Material mit hoher Dichte (> 4.5 g/cm³). Es kommen hierfür Metalle wie z.B. Titan in Betracht.
Der zweite und dritte Referenzkörper bestehen aus Materialien mittlerer Dichte (0.4-4.5 g/cm³). Keramik wie Calciumphosphat bzw. Polymere wie Polyethylen kommen hier in Betracht.
Als Material niedriger Dichte (< 0.4 g/cm³) dient das Trägermaterial der Vorrichtung (z. B. Glas oder Plexiglas).
Die Referenzkörper sind nebeneinander aufgereiht, so daß zwischen ihnen jeweils ein gewisser Abstand besteht. Insgesamt sind die Referenzkörper in dieser Anordnung in einem transparenten Formkörper aus Kunstharz fixiert. Vorzugsweise ist der Formkörper kubisch gestaltet, um von allen Seiten die gleiche Oberfläche im Strahlengang zu gewährleisten. Er kann aber auch anders gestaltet sein, z.B. in Form von Quadern, Rhomboedern, Prismen, Zylindern.
Die erfindungsgemäße Vorrichtung entspricht aufgrund ihrer Homogenität und der Kenntnisse über jede ihrer Komponenten ohne Einschränkungen dem Goldstandard.
Je nach Anwendungszweck finden im Kunstharzblock 2 oder mehr Referenzkörper Platz.

Gegenstand der Erfindung ist auch das Verfahren zur Durchführung der semiquantitativen Dichtebestimmung.
Dazu wird der Formkörper mit einem strahlendurchlässigen Klebestreifen auf die äußere Haut, in die Mitte der zu untersuchenden Zone, gebracht, so daß es auf dem Röntgenbild in der zu untersuchenden Zone zu sehen ist. Seine Ausrichtung muß so erfolgen, daß alle drei Materialien bzw. Referenzkörper aus denen sich der Formkörper zusammensetzt, zu sehen sind.
Der Formkörper ist je nach Typ des Röntgengerätes sowohl bei der klassischen Technik als auch bei der Tomodensitometrie (Scanner) einsetzbar.

Abhängig hiervon unterliegt die Positionierung des Formkörpers verschiedenen Regeln:
- klassische Technik:
   Der Formkörper muß vollständig auf dem Röntgenbild erscheinen, er überlagert die angrenzenden bzw. unter ihr liegenden Knochenstrukturen.
- Tomodensitometrie:
   Bei dieser Art der Röntgenaufnahme ist es notwendig, den Formkörper für jeden einzelnen Schnitt neu zu orientieren, so daß seine drei Elemente (Referenzkörper) vollständig integriert sind. Ein Probedurchlauf ist unumgänglich, um den Formkörper auf die zu untersuchende Knochenstruktur bzw. das zu untersuchende Material einzustellen.

Die Gebrauchsbedingungen wie der Typ des Filmes, der Apparatetyp (Dauer der Belichtung, Intensität etc.) oder die Art der Entwicklung (Bad, Temperatur etc.) sind identisch mit dem normalen (klassischen) Verfahren. Es ist nicht notwendig, andere Änderungen vorzunehmen als die Positionierung der Vorrichtung.
Um ein korrektes Ablesen zu gewährleisten, muß die gesamte Software zur Bildanalyse vorher einer Vergleichstudie über die Vorrichtung als feststehenes Bezugsobjekt unterzogen werden.

### Das Einbringen des Referenzkörpers in einen transparenten Kunstharzblock:

Man stellt in einem Gefäß eine Grundlage aus Kunstharz (Plexiglas) her, so daß der Boden vollständig mit dem Hart bedeckt ist.

Danach setzt man die Referenzkörper auf das Hart und richtet ihre Position mit einer Richtleiste aus. Mit Hilfe der Richtleiste können sie parallel ausgerichtet und der Abstand zwischen ihnen gemessen werden.

Nun gießt man den Rest des Harzes hinzu und läßt das Ganze polymerisieren.

Man erhält einen Kunstharzrohling mit einem Rauminhalt von 1 bis 10 cm³, vorzugsweise 2 cm³, der geschliffen werden muß, um hindurchschauen und die Plazierung der Referenzkörper erkennen zu können.
Danach wird der transparente Kunstharzblock auf eine Fräsmaschine montiert, um die Seiten in die erforderliche Ausrichtung zu bringen. Man sollte sich dabei die beste Position in bezug auf die Kanten des Referenzkörpers markieren. Die Bearbeitung sollte sehr vorsichtig erfolgen, um ein Zerbrechen der spröden Oberfläche des Harzblockes zu vermeiden.
Der Kunstharzblock, der nun in der gewünschten Ausrichtung ist, wird noch einmal poliert, um seine Transparenz zu gewähren.

Erläuterungen zu den Figuren 1 und 2:
- Fig.1:: Ansicht des Formkörpers von schräg oben.
- Fig.2:: Draufsicht mit Abmessungen in mm.

## Patentansprüche

1. Formkörper mit mindestens zwei parallelen Oberflächen zur semiquantitativen Dichtebestimmung von Knochen, Knochenersatzmaterial und Knochenimplantaten, dadurch gekennzeichnet, daß der Formkörper aus drei oder mehr verschiedenen Materialien mit unterschiedlichen Dichten besteht.

2. Formkörper nach Anspruch 1, dadurch gekennzeichnet, daß er zwei oder mehr Referenzkörper enthält.

3. Formkörper nach einem der Ansprüche 1 und/oder 2, dadurch gekennzeichnet, daß das Material
- hoher Dichte eine Dichte > 4.5 g/cm³
- mittlerer Dichte eine Dichte von 0.4-4.5 g/cm³
- niedriger Dichte eine Dichte < 0.4 g/cm³ aufweist.

4. Formkörper gemäß Anspruch 1-3, dadurch gekennzeichnet, daß das Material
- hoher Dichte aus Metallen wie Titan
- mittlerer Dichte aus Polymeren wie Polyethylen und/ oder Keramik wie Calciumphosphat
- niedriger Dichte aus Glas oder Plexiglas besteht.

5. Formkörper nach einem der Ansprüche 1 bis 4, dadurch gekennzeichnet, daß der Rauminhalt von 1 bis 10 cm³ beträgt.

6. Formkörper zur semiquantitativen Dichtebestimmung von Knochen, Knochenersatzmaterial und Knochenimplantaten mittels einem Formkörper nach einem der Ansprüche 1 bis 5, dadurch gekennzeichnet, daß
- der Formkörper mit einem strahlendurchlässigem Klebestreifen auf die äußere Haut, in die Mitte der zu untersuchenden Zone, gebracht,
- die Ausrichtung des Formkörpers so erfolgt, daß alle drei Elemente (Materialien) aus denen sich der Formkörper zusammensetzt, sichtbar sind,
- und eine Röntgenaufnahme mittels klassischer Technik erstellt wird.

7. Verfahren nach Anspruch 6, dadurch gekennzeichnet, daß die Röntgenaufnahme mittels Tomodensitometrie (Scanner) erstellt wird.

8. Verwendung des Formkörpers nach einem der Ansprüche 1 bis 5 zum Kalibrieren von Bildanalysesystemen bei der semiquantitativen Dichtebestimmung von Knochen, Knochenersatzmaterial und Knochenimplantaten.
